# FASCICULE DE BREVET EUROPEEN

(11) **EP 2 041 298 B1**
(45) Date de publication et mention de la délivrance du brevet: **22.12.2010**
(21) Numéro de dépôt: 07787282.8
(22) Date de dépôt: 10.07.2007
(51) Int. Cl.: C12Q 1/04, C12Q 1/10

(54) **MILIEU DE CULTURE SOLIDE POUR LA DETECTION ET/OU LA DISCRIMINATION AU NIVEAU DE L'ESPECE DES ENTEROCOQUES RESISTANTS AUX GLYCOPEPTIDES**
FESTES KULTURMEDIUM ZUM NACHWEIS UND/ODER DER ARTENUNTERSCHEIDUNG GLYKOPEPTID-RESISTENTER ENTEROKOKKEN
SOLID CULTURE MEDIUM FOR THE DETECTION AND/OR THE SPECIES DISCRIMINATION OF GLYCOPEPTIDE-RESISTANT ENTEROCOCCI

(30) Priorité: 10.07.2006 FR 0606252
(43) Date de publication de la demande: 01.04.2009
(73) Titulaire: Rambach, Alain, F-75006 Paris (FR)
(72) Inventeur: Rambach, Alain, F-75006 Paris (FR)
(74) Mandataire: Warcoin, Jacques
(86) Numéro de dépôt international: PCT/EP2007/057006
(87) Numéro de publication internationale: WO 2008/006816

(56) Documents cités:
- WO-A-98/04674
- FR-A- 2 861 741
- FR-A1- 2 826 019
- US-A1- 2004 241 747
- EFTHYMIOU C J ET AL: "IMPROVED ISOLATION AND DIFFERENTIATION OF ENTEROCOCCI IN CHEESE" APPLIED MICROBIOLOGY, WASHINGTON, DC, US, vol. 28, no. 3, 1974, pages 417-422, XP002363135 ISSN: 0003-6919

## Description

La présente invention se rapporte au domaine de la microbiologie médicale et clinique. Plus particulièrement, l'invention s'intéresse aux moyens permettant de détecter et/ou discriminer des espèces bactériennes responsables d'infections qui, parce qu'elles sont résistantes à un ou plusieurs antibiotiques, rendent nécessaire l'adaptation des traitements thérapeutiques.

De manière plus précise, la présente invention concerne un milieu de culture solide pour la détection et/ou la discrimination, chez les entérocoques résistants aux glycopeptides, des groupes d'espèces *E. faecalis* et/ou *E. faecium* appartenant aux groupes de résistance aux glycopeptides VanA/VanB, et *E. gallinarum*/*E. casseliflavus* appartenant au groupe de résistance aux glycopeptides VanC, ledit milieu comprenant, dans un milieu de culture sélectif pour entérocoques, au moins un substrat chromogène de l'α-glucosidase et au moins un activateur de réaction colorée choisi parmi le méthyl-α-glucoside et le glucosyl-α-glucoside, ou leurs polymères.

Des souches d'entérocoques résistantes à la vancomycine (VRE) et, plus largement aux glycopeptides, ont été pour la première fois isolées en clinique dans les années 80. Depuis, l'incidence des VRE en milieu hospitalier n'a cessé de croître. Ces bactéries sont généralement responsables d'infections nosocomiales préoccupantes pour le personnel médical. De plus, l'émergence de VRE multirésistants, c'est-à-dire des entérocoques résistants non seulement à la vancomycine, mais également à un ou plusieurs antibiotiques non glycopeptidiques, représente un vrai enjeu thérapeutique pour les cliniciens. En effet, la multiplicité et la dissémination des caractères de résistance entre souches d'entérocoques ont rendu certaines d'entre elles invulnérables aux thérapies standard.

A la différence des mécanismes qui confèrent une résistance à la plupart des familles d'antibiotiques, la résistance aux glycopeptides ne dépend pas du produit d'un gène unique mais nécessite l'expression régulée et interactive d'un ensemble de gènes étroitement associés : les opérons de résistance aux glycopeptides *van.*

A ce jour, plusieurs types (ou groupes) de résistance aux glycopeptides ont été identifiés chez les entérocoques, parmi lesquels les types VanA (opéron *vanA*)*,* VanB (opéron *vanB*) et VanC (opéron *vanC*) sont les plus fréquemment rencontrés (voir Tableau 1 ci-dessous). Certains de ces groupes de gènes sont transmissibles entre souches bactériennes (on parle de résistance acquise), alors que d'autres sont intrinsèques et non transmissibles (Tableau 1).

En définitive, au sein des VRE, les espèces d'entérocoques les plus fréquentes peuvent généralement être réparties en espèces ou groupes d'espèces plus spécifiquement associé(e)s à un type de résistance aux glycopeptides donné (Tableau 1).

**Tableau I**

| Type de résistance aux glycopeptides | Espèces d'entérocoques | Origine des gènes de résistance |
|---|---|---|
| VanA | *E. faecium,* *E. faecalis* | acquise |
| VanB | *E. faecium,* *E. faecalis* | acquise |
| VanC | *E. gallinarum,* *E. casseliflavus* | intrinsèque |

Comme le montre le Tableau II ci-dessous, les types de résistance aux glycopeptides chez les entérocoques se distinguent non seulement par les séquences génétiques responsables (séquences des opérons van), leur support (plasmidique ou chromosomique) et les mécanismes d'expression et de régulation de l'expression mis en jeu (résistance inductible ou constitutive), mais aussi par les niveaux de résistance aux glycopeptides associés.

**Tableau II**

| Type de résistance aux glycopeptides | CMI | |
|---|---|---|
| | Vancomycine | Teicoplanine |
| VanA | 64 -> 1024 | 16 -> 512 |
| VanB | 4 -> 1024 | 0,5 - 1 |
| VanC | 2 -> 32 | 0,5 - 1 |

| | | |
|---|---|---|
| CMI : concentration minimale inhibitrice en mg/l | | |

Afin de détecter et/ou discriminer les bactéries, on utilise communément des méthodes fondées sur la recherche de caractères soit phénotypiques, soit génotypiques. Plusieurs de ces méthodes peuvent d'ailleurs être avantageusement combinées pour augmenter le pouvoir discriminant de l'analyse, en améliorant la sélectivité et/ou la sensibilité et/ou la spécificité de la détection.

Dans le domaine particulier de la détection des VRE, une méthode phénotypique connue permet de discriminer, par coloration spécifique, les cultures de VRE VanC et les cultures de VRE non-VanC (qui ne se colorent pas). Cette méthode, mise en oeuvre en milieu liquide (tube ou puits), utilise une acidification du milieu liée à la présence de méthyl-α-glucoside. Dans ce test, le sucre est utilisé à une concentration de 20 g/l, en présence d'un indicateur de pH servant à révéler l'acidification du milieu.

Toutefois, le pouvoir discriminant de ce test demande encore à être amélioré, tant en termes de sélectivité que de spécificité, et ce, d'une part afin d'éliminer les résultats faux-positifs ou faux-négatifs susceptibles d'être obtenus, d'autre part afin de permettre la détection des VRE au niveau de l'espèce, ce qui est impossible avec le test actuel.

Le brevet FR2861741 divulgue un milieu de culture pour détecter un Entérocoque caractérisé en ce qu'il comprend un réactif chromogène réducteur qui donne une coloration avec *E. faecalis* mais ne donne pas de coloration avec *E*. *faecium.*

Il existe donc actuellement un besoin pour un test de détection des VRE les plus fréquemment rencontrés en clinique, qui soit à la fois simple, rapide, sensible, sélectif, spécifique et fiable, pour permettre la détection discriminante des VRE au niveau de l'espèce et au niveau du type de résistance aux glycopeptides associé.

C'est précisément à ce besoin que répond la présente invention en proposant des moyens phénotypiques utilisables en milieu solide (sur boîtes) pour la détection efficace et discriminante des VRE.

A cet égard, on notera que, comme le montrent l'exemple et le Tableau III ci-dessous, la technique du test en tube de l'état de la technique, si elle est appliquée telle quelle en milieu solide, ne conduit qu'à peu de coloration des colonies, ce qui ne permet donc pas de discriminer ou différencier les VRE.

Par ailleurs, il est ici montré (voir Tableau III infra) qu'un substrat chromogène utilisé en milieu solide ne permet pas, à lui seul, de détecter les VRE de manière satisfaisante.

Les résultats rapportés dans le cadre de l'invention montrent que la combinaison en tube du méthyl-α-glucoside et d'un indicateur de pH ne peut pas être simplement remplacée, en milieu solide, par un substrat chromogène de l'α-glucosidase, contrairement à ce que l'homme du métier aurait pressenti en partant de l'hypothèse vraisemblable que la détection se faisait par simple mise en évidence de la présence ou de l'absence du gène codant l'enzyme α-glucosidase.

En outre, la présente invention montre que des combinaisons différentes de deux substrats au moins de l'α-glucosidase, dont l'un est chromogène, conduisent, en milieu solide, à des résultats tout à fait inattendus dans la mesure où la coloration s'inverse en fonction des substrats mis en oeuvre et de leurs combinaisons. On notera d'ailleurs que des substrats de l'α-glucosidase autres que ceux explicitement décrits ci-dessous, ont été testés, sans succès, par l'Inventeur. Par exemple, contrairement au maltose visé ci-dessous, le tréhalose ne permet pas d'obtenir des résultats intéressants en termes de détection et différenciation des espèces ou groupes d'espèces de VRE (données non montrées).

Finalement, les résultats obtenus dans le cadre de l'invention sont non seulement surprenants et inattendus, mais aussi particulièrement avantageux car ils permettent de détecter de manière discriminante les VRE VanC par rapport aux VRE VanA/VanB (et réciproquement), ou bien l'espèce *E. faecalis* ou encore l'espèce *E. faecium,* par rapport aux autres espèces de VRE.

Ainsi, un premier aspect de la présente invention concerne un milieu de culture solide pour la détection et/ou la discrimination des groupes d'espèces d'entérocoques résistants aux glycopeptides :
- *E. faecalis* et/ou *E*. *faecium,* appartenant aux groupes de résistance aux glycopeptides VanA/VanB ; et
- *E. gallinarum*/*E. casseliflavus* appartenant au groupe de résistance aux glycopeptides VanC,
ledit milieu comprenant, dans un milieu de culture sélectif pour entérocoques, au moins un substrat chromogène de l'α-glucosidase et au moins un activateur de réaction colorée choisi parmi le méthyl-α-glucoside ou ses polymères et le glucosyl-α-glucoside ou ses polymères.

L'utilisation, dans un milieu solide, d'un substrat chromogène de l'α-glucosidase associé à au moins un activateur de réaction colorée, telle que cela est décrit pour la première fois ici, présente notamment l'avantage de permettre l'élaboration de milieux solides qui rendent possible la différenciation des espèces ou groupes d'espèces de VRE dès l'isolement, sans avoir à effectuer de tests supplémentaires.

Par « polymères du méthyl-α-glucoside », on désigne ici les oligosides et polyosides adaptés à une utilisation dans un milieu de culture bactérienne solide et susceptibles de libérer, après rupture des liaisons osidiques (par exemple, par dégradation enzymatique), le méthyl-α-glucoside. Un exemple typique d'un tel polymère est le méthyl-α-maltoside.

Par « polymères du glucosyl-α-glucoside », on désigne ici les oligosides et polyosides adaptés à une utilisation dans un milieu de culture bactérienne solide et susceptibles de libérer, après rupture des liaisons osidiques (par exemple, par dégradation enzymatique), le glucosyl-α-glucoside, plus communément appelé maltose.

Un « activateur de réaction colorée » est, au sens de la présente invention, un composé qui a pour fonction d'améliorer la qualité de la réaction observée. Ce composé est choisi parmi le méthyl-α-glucoside ou ses polymères et le glucosyl-α-glucoside ou ses polymères. Comme il ressort des résultats détaillés dans le Tableau III (voir l'exemple ci-dessous), la présence d'au moins un activateur de réaction colorée dans le milieu de culture solide selon la présente invention est essentielle pour détecter de manière discriminante les espèces ou groupes d'espèces de VRE.

La présente invention rend ainsi possible la détection et, avantageusement, l'identification au niveau de l'espèce ou du groupe d'espèces, une colonie colorée de VRE, même en présence de milliers d'autres colonies susceptibles de se développer sur le milieu sélectif solide objet de l'invention. En fait, les colonies de VRE détectées par le milieu de l'invention sont colorées d'une certaine couleur tandis que les autres microorganismes apparaissent de couleurs différentes ou sont incolores.

En outre, la détection des colonies de VRE est, dans le cadre de la présente invention, directe en ce sens qu'elle ne nécessite aucun acte ou intervention, telle que l'utilisation d'un révélateur quelconque ou celle d'une lumière particulière, postérieurement à la culture des bactéries.

Selon un mode de réalisation, le milieu de culture objet de la présente invention comprend au moins un substrat chromogène de l'α-glucosidase et le méthyl-α-glucoside ou l'un de ses polymères, utilisé à titre d'activateur de réaction colorée à une concentration d'au moins 10 g/l environ, pour la détection et/ou la discrimination par coloration des espèces *E. gallinarum*/*E. casseliflavus* appartenant au groupe de résistance aux glycopeptides VanC. Dans ces conditions, c'est par la coloration bien particulière et spécifique des colonies de *E. ga*//*inarum*/*E. casseliflavus* que la différenciation est rendue possible. Cette couleur, amenée par le substrat chromogène, ne concernera que les colonies de *E. gallinarum*/*E. casseliflavus,* d'où la notion de spécificité de la coloration.

De manière préférée, la concentration du méthyl-α-glucoside dans le milieu est d'au moins 20 g/l environ. De préférence encore, cette concentration est de 20 g/l environ.

Selon un autre mode de réalisation, le milieu de culture de l'invention comprend au moins un substrat chromogène de l'α-glucosidase et le glucosyl-α-glucoside (maltose) ou l'un de ses polymères, utilisé à titre d'activateur de réaction colorée à une concentration d'au moins 0,1 g/l environ, pour la détection et/ou la discrimination par coloration de l'espèce *E. faecalis* appartenant aux groupes de résistance aux glycopeptides VanA/VanB. Ici, seules les colonies des souches de l'espèce *E. faecalis* sont de la couleur amenée par le substrat chromogène sur les boîtes.

De manière préférée, la concentration du maltose dans le milieu est d'au moins 0,2 g/l environ. De préférence encore, cette concentration est de 0,2 g/l environ.

Selon encore un autre mode de réalisation, le milieu de culture de l'invention comprend au moins un substrat chromogène de l'α-glucosidase et, à titre d'activateurs de réaction colorée, le méthyl-α-glucoside ou l'un de ses polymères, utilisé à une concentration d'au moins 10 g/l environ, et le glucosyl-α-glucoside ou l'un de ses polymères, utilisé à une concentration d'au moins 0,1 g/l environ, pour la détection et/ou la discrimination par l'absence de coloration de l'espèce *E. faecium* appartenant aux groupes de résistance aux glycopeptides VanA/VanB. Dans ce cas, les colonies des souches des espèces *E. faecalis* et *E. gallinarum*/*E. casselitlavus* sont colorées grâce au substrat chromogène sur les boîtes. Seules les colonies des souches de *E. faecium* ne sont pas colorées grâce à ce substrat, ce qui permet une différenciation qualifiée de différenciation « par défaut de coloration », c'est-à-dire par absence de la coloration amenée par le substrat chromogène, étant entendu que les colonies de *E. faecium* pourront apparaître d'une couleur différente si un ou plusieurs autres composants colorants sont ajoutés dans le milieu.

De manière préférée, les concentrations du méthyl-α-glucoside et du maltose dans le milieu sont respectivement d'au moins 20 g/l environ et d'au moins 0,2 g/l environ. De préférence encore, ces concentrations sont respectivement de 20 g/l environ et 0,2 g/l environ.

Ainsi, selon les modes de réalisation mis en oeuvre, le milieu de culture objet de l'invention permet de discriminer aisément une espèce ou un groupe d'espèces de VRE, par simple coloration ou coloration différente ou encore absence de coloration des colonies présentes sur le milieu de culture solide.

Le « milieu de culture sélectif pour entérocoques » comprend divers facteurs sélectifs pour entérocoques, bien connus de l'homme du métier. Par exemple, des inhibiteurs des bactéries à Gram négatif permettent d'améliorer les propriétés du milieu de culture et d'isoler plus facilement les entérocoques (bactéries à Gram positif).

Il peut en outre être intéressant, afin d'optimiser les résultats donnés par le milieu de culture selon l'invention, de rajouter des facteurs connus comme étant sélectifs pour VRE ; ces facteurs permettront d'obtenir un milieu de culture solide spécifique pour VRE. De préférence, un tel milieu sélectif et spécifique pour VRE comprend au moins un antibiotique glycopeptidique.

Dans'un mode de réalisation, ledit antibiotique glycopeptidique est choisi parmi la vancomycine, la teicoplanine et leurs combinaisons.

Avantageusement, la vancomycine est utilisée à une concentration allant de 2 à 15 mg/l environ, de préférence à une concentration allant de 3 à 9 mg/l environ, de préférence encore à une concentration allant de 4 à 8 mg/l environ, avec une valeur particulièrement préférée à 6 mg/l environ.

La teicoplanine est, quant à elle, utilisée à une concentration allant de 0,5 à 16 mg/l environ, de préférence à une concentration allant de 1 à 10 mg/l environ, de préférence encore à une concentration allant de 2 à 8 mg/l environ, avec une valeur particulièrement préférée à 5 mg/l environ.

Bien entendu, l'homme du métier est en tous les cas capable de déterminer et, si nécessaire, tester les concentrations ou gammes de concentrations du ou des glycopeptide(s), appropriées pour mettre en oeuvre la présente invention (d'après ses connaissances générales, en recherchant dans la littérature et/ou en se fondant sur le Tableau II qui précède)..

L'agent chromogène substrat de l'α-glucosidase comprend de préférence un chromophore précipitable, qui est libéré par l'hydrolyse du substrat par son enzyme. Ainsi, la colonie bactérienne se colore en fonction du chromophore libéré. Le chromophore libéré étant solide dans le milieu de culture, il reste donc localisé au niveau de la colonie où il a été libéré.

De façon préférée, ledit chromophore est choisi parmi les dérivés indoxyle, halogéno-indoxyle (bromo-indoxyle, chloro-indoxyle, fluoro-indoxyle, iodo-indoxyle, dichloro-indoxyle, chloro-bromo-indoxyle, tri-chloro-indoxyle), le méthyl-indoxyle, ou hydroxy-quinoline. Des dérivés particulièrement préférés sont choisis parmi les dérivés suivants : 6-chloro-indoxyle, 5-bromo-indoxyle, 3-bromo-indoxyle, 6-fluoro-indoxyle, 5-iodo-indoxyle, 4,6-dichloro-indoxyle, 6,7-dichloro-indoxyle, 5-bromo-4-chloro-indoxyle, 5-bromo-6-chloro-indoxyle, 4,6,7-trichloro-indoxyle, N-méthyl-indoxyle et 8-hydroxy-quinoline.

Plus préférentiellement, le substrat chromogène de l'α-glucosidase est un 5-bromo-4-chloro-indoxyl-α-glucoside.

Le substrat chromogène est typiquement utilisé à une concentration allant de 0,01 à 0,5 g/l environ. Une concentration préférée se situe à 0,1 g/l environ.

Il est précisé que le substrat chromogène présent dans le milieu de culture selon la présente invention peut être remplacé, ou associé, à au moins un substrat fluorogène. Ce substrat est formé par couplage d'un substrat de l'α-glucosidase avec un fluorophore tel que le 4-méthyl umbelliféryl.

Un deuxième aspect de la présente invention concerne l'utilisation d'un milieu de culture tel que défini ci-dessus, pour la détection et/ou la discrimination des groupes d'espèces d'entérocoques résistants aux glycopeptides :
- *E. faecalis* et/ou *E*. *faecium,* appartenant aux groupes de résistance aux glycopeptides VanA/VanB ; et
- E. *gallinarum*/*E. casseliflavus* appartenant au groupe de résistance aux glycopeptides VanC.

Un troisième aspect de l'invention concerne un procédé de détection et/ou discrimination, dans un échantillon, des groupes d'espèces d'entérocoques résistants aux glycopeptides :
- *E. faecalis* et/ou *E. faecium,* appartenant aux groupes de résistance aux glycopeptides VanA/VanB ; et
- *E. gallinarum*/*E. casseliflavus* appartenant au groupe de résistance aux glycopeptides VanC,
caractérisé en ce qu'il comprend au moins les étapes suivantes :
a) l'inoculation d'un milieu de culture tel que défini ci-dessus, avec ledit échantillon ou un inoculum dérivé de celui-ci ;
b) la détection, sur ledit milieu de culture, de la présence de groupes d'espèces d'entérocoques résistants aux glycopeptides *E. faecalis*/*E. faecium* et *E. gallinarum*/*E. casseliflavus ;* et
c) de façon optionnelle, la différenciation des espèces *E. faecalis* et/ou *E. faecium* et/ou *E. gallinarum*/*E. casseliflavus,* des autres microorganismes présents sur ledit milieu de culture.

L'utilisation du milieu de culture conforme à l'invention peut être avantageusement précédée d'une étape d'enrichissement réalisée à l'aide de méthodes connues de l'homme du métier. On peut utiliser en particulier de l'eau peptonée alcaline à 1 g % de NaCl (pH 8,6), ou de l'eau peptonée alcaline à 3 g % de NaCl.

L'objet de la présente invention ne se limite pas à la description ci-dessus. D'autres modes de réalisation et avantages de la présente invention pourront ressortir de l'exemple ci-dessous, fourni ici à titre purement illustratif. Il est clair que ni l'objet ni la portée de la présente invention ne sont limités par cet exemple.

### EXEMPLE

### a) Base du milieu de culture solide (en g/l) :

- Peptone 10
- Extrait de levure 5
- Agar 15

### b) Glycopeptides :

Plusieurs séries d'expériences ont été réalisées : certaines séries ont été mises en oeuvre sur un milieu sélectif pour entérocoques (dépourvu de glycopeptides), d'autres sur un milieu sélectif pour VRE, contenant 6 mg/l de vancomycine. Les résultats rapportés dans le Tableau III ci-dessus reflètent les résultats moyens obtenus sur l'ensemble des séries.

### c) Autres ingrédients (en g/l) :

Voir le Tableau III ci-après.
X-α-glucoside : substrat chromogène de l'α-glucosidase.

Les résultats rapportés dans le Tableau III suivant ont été obtenus après ensemencement des bactéries sur les milieux de culture indiqués, suivi dans tous les cas d'une incubation de 24 heures environ à 37°C environ.

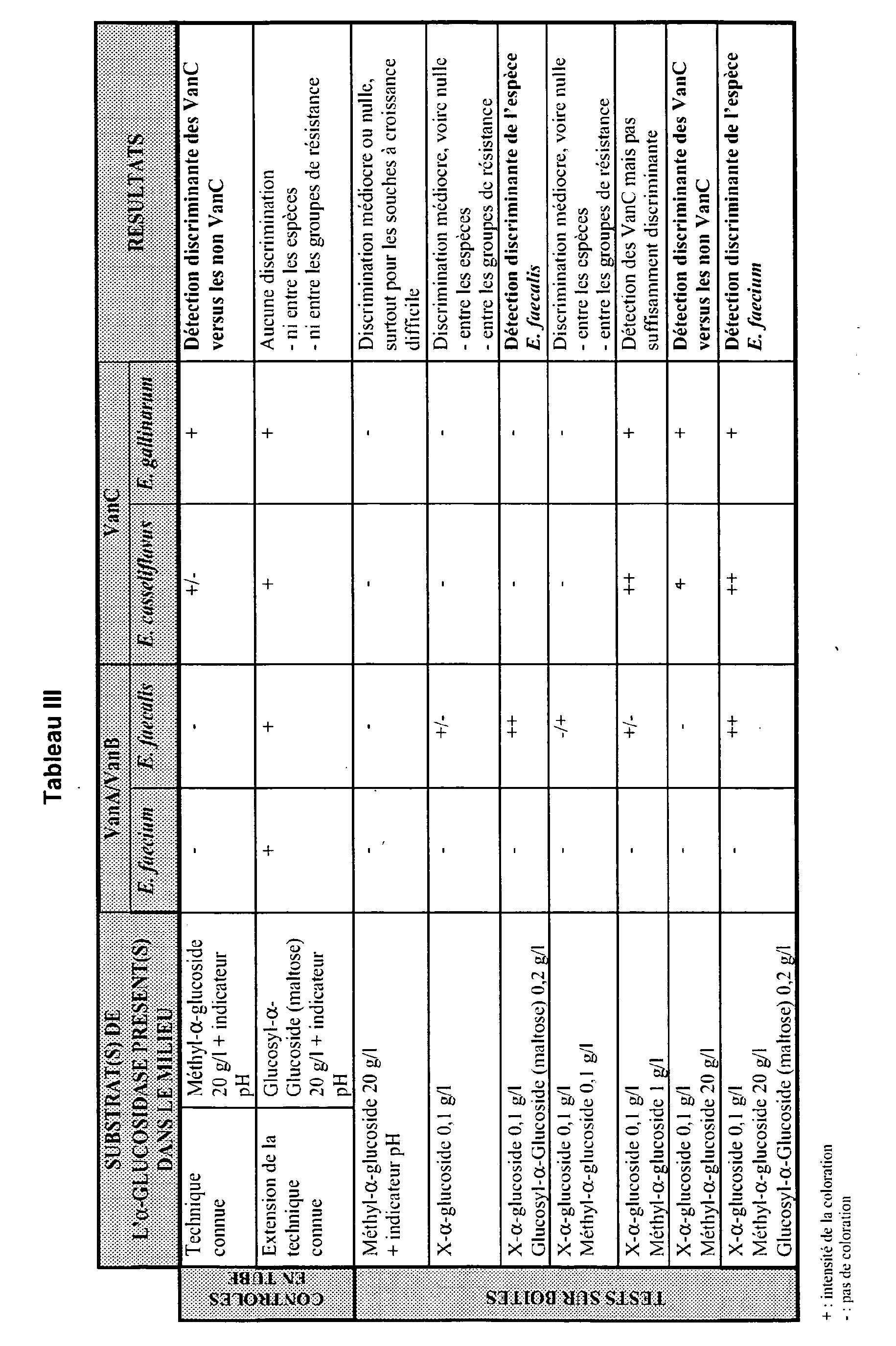

## Revendications

1. Milieu de culture solide pour la détection et/ou la discrimination des groupes d'espèces d'entérocoques résistants aux glycopeptides :
- *E. faecalis* et/ou *E. faecium,* appartenant aux groupes de résistance aux glycopeptides VanA/VanB ; et
- *E. gallinarum*/*E. casseliflavus* appartenant au groupe de
résistance aux glycopeptides VanC,
ledit milieu comprenant, dans un milieu de culture sélectif pour entérocoques, au moins un substrat chromogène de l'α-glucosidase et au moins un activateur de réaction colorée choisi parmi le méthyl-α-glucoside ou ses polymères et le glucosyl-α-glucoside ou ses polymères.

2. Milieu de culture selon la revendication 1, **caractérisé en ce qu'**il comprend au moins un substrat chromogène de l'α-glucosidase et le méthyl-α-glucoside ou l'un de ses polymères, utilisé à une concentration d'au moins 10 g/l environ, pour la détection et/ou la discrimination des espèces *E. gallinarum*/*E. casseliflavus* appartenant au groupe de résistance aux glycopeptides VanC.

3. Milieu de culture selon la revendication 1, **caractérisé en ce qu'**il comprend au moins un substrat chromogène de l'α-glucosidase et le glucosyl-α-glucoside ou l'un de ses polymères, utilisé à une concentration d'au moins 0,1 g/l environ, pour la détection et/ou la discrimination de l'espèce *E. faecalis* appartenant aux groupes de résistance aux glycopeptides VanA/VanB.

4. Milieu de culture selon la revendication 1, **caractérisé en ce qu'**il comprend au moins un substrat chromogène de l'α-glucosidase, le méthyl-α-glucoside ou l'un de ses polymères, utilisé à une concentration d'au moins 10 g/l environ, et le glucosyl-α-glucoside ou l'un de ses polymères, utilisé à une concentration d'au moins 0,1 g/l environ, pour la détection et/ou la discrimination de l'espèce *E. faecium* appartenant aux groupes de résistance aux glycopeptides VanA/VanB.

5. Milieu de culture selon l'une quelconque des revendications 1 à 4, **caractérisé en ce que** ledit milieu de culture sélectif pour entérocoques est un milieu sélectif pour entérocoques résistants aux glycopeptides comprenant au moins un antibiotique glycopeptidique.

6. Milieu de culture selon la revendication 5, **caractérisé en ce que** ledit antibiotique glycopeptidique est choisi parmi la vancomycine, la teicoplanine et leurs combinaisons.

7. Milieu de culture selon la revendication 6, **caractérisé en ce que** la vancomycine est utilisée à une concentration allant de 2 à 15 mg/l environ, de préférence à une concentration allant de 3 à 9 mg/l environ, de préférence encore à une concentration allant de 4 à 8 mg/l environ, avec une valeur particulièrement préférée à 6 mg/l environ

8. Milieu de culture selon la revendication 6, **caractérisé en ce que** la teicoplanine est utilisée à une concentration allant de 0,5 à 16 mg/l environ, de préférence à une concentration allant de 1 à 10 mg/l environ, de préférence encore à une concentration allant de 2 à 8 mg/l environ, avec une valeur particulièrement préférée à 5 mg/l environ.

9. Milieu de culture selon l'une quelconque des revendications 1 à 8, **caractérisé en ce que** ledit substrat chromogène libère, par hydrolyse, un chromophore précipitable choisi parmi les dérivés indoxyle, halogéno-indoxyle (bromo-indoxyle, chloro-indoxyle, fluoro-indoxyle, iodo-indoxyle, dichloro-indoxyle, chloro-bromo- indoxyle, tri-chloro-indoxyle), méthyl-indoxyle, ou hydroxy-quinoline, en particulier les dérivés suivants : 6-chloro-indoxyle, 5-bromo-indoxyle, 3-bromo-indoxyle, 6- fluoro-indoxyle, 5-iodo-indoxyle, 4,6-dichloro-indoxyle, 6,7-dichloro-indoxyle, 5-bromo-4-chloro-indoxyle, 5-bromo-6-chloro-indoxyle, 4,6,7-trichloro-indoxyle, N-méthyl-indoxyle ou 8-hydroxy-quinoline.

10. Milieu de culture selon l'une quelconque des revendications 1 à 9, **caractérisé en ce que** ledit substrat chromogène de l'α-glucosidase est un 5-bromo-4-chloro-indoxyl-α-glucoside.

11. Utilisation d'un milieu de culture selon l'une quelconque des revendications 1 à 10, pour la détection et/ou la discrimination des groupes d'espèces d'entérocoques résistants aux glycopeptides :
- *E. faecalis* et/ou *E. faecium,* appartenant aux groupes de résistance aux glycopeptides VanA/VanB ; et
- *E. gallinarum*/*E. casselitlavus* appartenant au groupe de résistance aux glycopeptides VanC.

12. Procédé de détection et/ou discrimination, dans un échantillon, des groupes d'espèces d'entérocoques résistants aux glycopeptides :
- *E*. *faecalis* et/ou *E. faecium,* appartenant aux groupes de résistance aux glycopeptides VanA/VanB ; et
- *E. gallinarum*/*E. casseliflavus* appartenant au groupe de résistance aux glycopeptides VanC,
**caractérisé en ce qu'**il comprend au moins les étapes suivantes :
a) l'inoculation d'un milieu de culture tel que défini dans l'une quelconque des revendications 1 à 10 avec ledit échantillon ou un inoculum dérivé de celui-ci ;
b) la détection, sur ledit milieu de culture, de la présence de groupes d'espèces d'entérocoques résistants aux glycopeptides *E. faecalis*/*E. faecium* et *E. gallinarum*/*E. casseliflavus ;* et
c) de façon optionnelle, la différenciation des espèces *E. faecalis* et/ou *E. faecium* et/ou *E. gallinarum*/*E. casseliflavus,* des autres microorganismes présents sur ledit milieu de culture.

## Claims

1. A solid culture medium for the detection and/or the discrimination of groups of glycopeptide-resistant species of enterococci:
- *E. faecalis* and/or *E. faecium,* belonging to the VanA/VanB groups of resistance to glycopeptides; and
- *E. gallinarum*/*E. casseliflavus,* belonging to the VanC group of resistance to glycopeptides,
wherein said medium comprises, in a culture medium selective for enterococci, at least one chromogenic substrate for α-glucosidase and at least one activator of a colored reaction chosen from methyl-α-glucoside or polymers thereof and glucosyl-α-glucoside or polymers thereof.

2. The culture medium of claim 1, **characterized in that** it comprises at least one chromogenic substrate for α-glucosidase and methyl-α-glucoside or a polymer thereof, used at a concentration of at least 10 g/l approximately, for the detection and/or the discrimination of the species *E. gallinarum*/*E. casseliflavus* belonging to the VanC group of resistance to glycopeptides.

3. The culture medium of claim 1, **characterized in that** it comprises at least one chromogenic substrate for α-glucosidase and glucosyl-α-glucoside or a polymer thereof, used at a concentration of at least 0.1 g/l approximately, for the detection and/or the discrimination of the species *E. faecalis* belonging to the VanA/VanB groups of resistance to glycopeptides.

4. The culture medium of claim 1, **characterized in that** it comprises at least one chromogenic substrate for α-glucosidase, methyl-α-glucoside or a polymer thereof, used at a concentration of at least 10 g/l approximately, and glucosyl-α-glucoside or a polymer thereof, used at a concentration of at least 0.1 g/l approximately, for the detection and/or the discrimination of the species *E. faecium* belonging to the VanA/VanB groups of resistance to glycopeptides.

5. The culture medium according to any of claims 1 to 4, **characterized in that** said culture medium selective for enterococci is a medium selective for glycopeptide-resistant enterococci comprising at least one glycopeptide antibiotic.

6. The culture medium of claim 5, **characterized in that** said glycopeptide antibiotic is chosen from vancomycin, teicoplanin and combinations thereof.

7. The culture medium of claim 6, **characterized in that** vancomycin is used at a concentration from 2 mg/l to 15 mg/l approximately, preferably at a concentration from 3 mg/l to 9 mg/l approximately, more preferably at a concentration from 4 mg/l to 8 mg/l approximately, with a particularly preferred value of 6 mg/l approximately.

8. The culture medium of claim 6, **characterized in that** teicoplanin is used at a concentration from 0.5 mg/l to 16 mg/l approximately, preferably at a concentration from 1 mg/l to 10 mg/l approximately, more preferably at a concentration from 2 mg/l to 8 mg/l approximately, with a particularly preferred value of 5 mg/l approximately.

9. The culture medium according to any of claims 1 to 8, **characterized in that** said chromogenic substrate releases, by hydrolysis, a precipitable chromophore chosen from the indoxyl, halogen indoxyl (bromo-indoxyl, chloro-indoxyl, fluoro-indoxyl, iodo-indoxyl, dichloro-indoxyl, chloro-bromo-indoxyl, trichloro-indoxyl), methyl-indoxyl or hydroxy-quinoline derivatives, in particular the following derivatives: 6-chloro-indoxyl, 5-bromo-indoxyl, 3-bromo-indoxyl, 6-fluoro-indoxyl, 5-iodo-indoxyl, 4,6-dichloro-indoxyl, 6,7-dichloro-indoxyl, 5-bromo-4-chloro-indoxyl, 5-bromo-6-chloro-indoxyl, 4,6,7-trichloro-indoxyl, N-methyl-indoxyl or 8-hydroxy-quinoline.

10. The culture medium according to any of claims 1 to 9, **characterized in that** said chromogenic substrate for α-glucosidase is a 5-bromo-4-chloro-indoxyl-α-glucoside.

11. Use of the culture medium according to any of claims 1 to 10, for the detection and/or the discrimination of the groups of glycopeptide-resistant species of enterococci:
- *E. faecalis* and/or *E. faecium,* belonging to the VanA/VanB groups of resistance to glycopeptides; and
- *E. gallinarum*/*E. casseliflavus,* belonging to the VanC group of resistance to glycopeptides.

12. A method for the detection and/or the discrimination, in a sample, of groups of glycopeptide-resistant species of enterococci:
- *E. faecalis* and/or *E. faecium,* belonging to the VanA/VanB groups of resistance to glycopeptides; and
- *E. gallinarum*/*E. casseliflavus,* belonging to the VanC group of resistance to glycopeptides,
**characterized in that** it comprises at least the following steps:
a) inoculation of a culture medium as defined in any of claims 1 to 10 with said sample or an inoculum derived therefrom;
b) detection, on said culture medium, of the presence of groups of glycopeptide-resistant species of enterococci *E. faecalis*/*E. faecium* and *E. gallinarum*/*E. casseliflavus;* and
c) optionally, differentiation of the species *E*. *faecalis* and/or *E. faecium* and/or *E. gallinarum*/*E. casseliflavus* from other microorganisms present on said culture medium.

## Patentansprüche

1. Festes Kulturmedium zum Nachweis und/oder zur Unterscheidung von Gruppen von Spezies von gegen Glykopeptide resistenten Enterokokken:
- *E. faecalis* und/oder *E. faecium,* die zu den Gruppen mit Resistenz gegen VanA/VanB-Glykopeptide gehören; und
- *E. gallinarium*/*E. casseliflavus,* die zu der Gruppe mit Resistenz gegen VanC-Glykopeptide gehören,
wobei das Medium in einem für Enterokokken selektiven Kulturmedium mindestens ein chromogenes α-Glucosidase-Substrat und mindestens einen Aktivator der Farbreaktion umfasst, der aus Methyl-α-glucosid oder seinen Polymeren und Glucosyl-α-glucosid oder seinen Polymeren ausgewählt ist.

2. Kulturmedium nach Anspruch 1, **dadurch gekennzeichnet, dass** es mindestens ein chromogenes α-Glucosidase-Substrat und Methyl-α-glucosid oder eines seiner Polymere, das in einer Konzentration von mindestens etwa 10 g/l verwendet wird, zum Nachweis und/oder zur Unterscheidung der Spezies *E. gallinarium*/*E. casseliflavus,* die zu der Gruppe mit Resistenz gegen VanC-Glykopeptide gehören, umfasst.

3. Kulturmedium nach Anspruch 1, **dadurch gekennzeichnet, dass** es mindestens ein chromogenes α-Glucosidase-Substrat und Glucosyl-α-glucosid oder eines seiner Polymere, das in einer Konzentration von mindestens etwa 0,1 g/l verwendet wird, zum Nachweis und/oder zur Unterscheidung der Spezies *E. faecalis,* die zu den Gruppen mit Resistenz gegen VanA/VanB-Glykopeptide gehört, umfasst.

4. Kulturmedium nach Anspruch 1, **dadurch gekennzeichnet, dass** es mindestens ein chromogenes α-Glucosidase-Substrat, Methyl-α-glucosid oder eines seiner Polymere, das in einer Konzentration von mindestens etwa 10 g/l verwendet wird, und Glucosyl-α-glucosid oder eines seiner Polymere, das in einer Konzentration von mindestens etwa 0,1 g/l verwendet wird, zum Nachweis und/oder zur Unterscheidung der Spezies *E. faecium,* die zu den Gruppen mit Resistenz gegen VanA/VanB-Glykopeptide gehört, umfasst.

5. Kulturmedium nach irgendeinem der Ansprüche 1 bis 4, **dadurch gekennzeichnet, dass** das für Enterokokken selektive Kulturmedium ein für gegen Glykopeptide resistente Enterokokken selektives Medium ist, das mindestens ein Glykopeptid-Antibiotikum umfasst.

6. Kulturmedium nach Anspruch 5, **dadurch gekennzeichnet, dass** das Glykopeptid-Antibiotikum aus Vancomycin, Teicoplanin und deren Kombinationen ausgewählt ist.

7. Kulturmedium nach Anspruch 6, **dadurch gekennzeichnet, dass** das Vancomycin in einer Konzentration von etwa 2 bis 15 mg/l, vorzugsweise einer Konzentration von etwa 3 bis 9 mg/l, bevorzugter in einer Konzentration von etwa 4 bis 8 mg/l, mit einem besonders bevorzugten Wert von etwa 6 mg/l, verwendet wird.

8. Kulturmedium nach Anspruch 6, **dadurch gekennzeichnet, dass** das Teicoplanin in einer Konzentration von etwa 0,5 bis 16 mg/l, vorzugsweise einer Konzentration von etwa 1 bis 10 mg/l, bevorzugter in einer Konzentration von etwa 2 bis 8 mg/l, mit einem besonders bevorzugten Wert von etwa 5 mg/l, verwendet wird.

9. Kulturmedium nach irgendeinem der Ansprüche 1 bis 8, **dadurch gekennzeichnet, dass** das chromogene Substrat mittels Hydrolyse ein ausfällbares Chromophor freisetzt, das aus den Indoxyl-, Halogenindoxyl- (Bromindoxyl-, Chlorindoxyl-, Fluorindoxyl-, Iodindoxyl-, Dichlorindoxyl-, Chlorbromindoxyl-, Trichlorindoxyl-), Methylindoxyl- oder Hydroxychinolin-Derivaten, insbesondere den folgenden Derivaten: 6-Chlorindoxyl, 5-Bromindoxyl, 3-Bromindoxyl, 6-Fluorindoxyl, 5-Iodindoxyl, 4,6-Dichlorindoxyl, 6,7-Dichlorindoxyl, 5-Brom-4-chlorindoxyl, 5-Brom-6-chlorindoxyl, 4,6,7-Trichlorindoxyl, N-Methylindoxyl oder 8-Hydroxychinolin, ausgewählt ist.

10. Kulturmedium nach irgendeinem der Ansprüche 1 bis 9, **dadurch gekennzeichnet, dass** das chromogene α-Glucosidase-Substrat ein 5-Brom-4-chlorindoxyl-α-glucosid ist.

11. Verwendung eines Kulturmediums nach irgendeinem der Ansprüche 1 bis 10 zum Nachweis und/oder zur Unterscheidung von Gruppen von Spezies von gegen Glykopeptide resistenten Enterokokken:
- *E. faecalis* und/oder *E. faecium,* die zu den Gruppen mit Resistenz gegen VanA/VanB-Glykopeptide gehören; und
- *E. gallinarium*/*E. casseliflavus,* die zu der Gruppe mit Resistenz gegen VanC-Glykopeptide gehören.

12. Verfahren zum Nachweis und/oder zur Unterscheidung von Gruppen von Spezies von gegen Glykopeptide resistenten Enterokokken:
- *E. faecalis* und/oder *E. faecium,* die zu den Gruppen mit Resistenz gegen VanA/VanB-Glykopeptide gehören; und
- *E. gallinarium*/*E. casseliflavus,* die zu der Gruppe mit Resistenz gegen VanC-Glykopeptide gehören,
in einer Probe,
**dadurch gekennzeichnet, dass** das Verfahren mindestens die folgenden Schritte umfasst:
a) Inokulation eines wie in irgendeinem der Ansprüche 1 bis 10 definierten Kulturmediums mit der Probe oder einem von dieser abgeleiteten Inokulum;
b) Nachweis der Anwesenheit von Gruppen von Spezies der gegen Glykopeptide resistenten Enterokokken *E. faecalis*/*E. faecium* und *E. gallinarium*/*E. casseliflavus* auf dem Kulturmedium; und
c) optional, Differenzierung der Spezies *E. faecalis* und/oder *E. faecium* und/oder *E. gallinarium*/*Ee. casseliflavus* von anderen auf dem Kulturmedium vorhandenen Mikroorganismen.
